# EUROPEAN PATENT SPECIFICATION

(11) **EP 3 885 369 B1**
(45) Date of publication and mention of the grant of the patent: **09.10.2024**
(21) Application number: 19931049.1
(22) Date of filing: 24.12.2019
(51) Int. Cl.: C07K 19/00, C12N 15/62, G01N 33/68

(54) **RECOMBINANT DEAMIDATED GLIADIN POLYPEPTIDE ANTIGEN, RECOMBINANT ANTIGEN EXPRESSION GENE, RECOMBINANT EXPRESSION VECTOR, PREPARATION METHOD THEREFOR, AND APPLICATION THEREOF**
REKOMBINANTES DEAMIDIERTES GLIADIN-POLYPEPTID-ANTIGEN, REKOMBINANTES ANTIGEN-EXPRESSIONSGEN, REKOMBINANTER EXPRESSIONSVEKTOR, HERSTELLUNGSVERFAHREN DAFÜR UND ANWENDUNG DAVON
ANTIGÈNE POLYPEPTIDIQUE DE TYPE GLIADINE DÉSAMIDÉE RECOMBINÉE, GÈNE D'EXPRESSION D'ANTIGÈNE RECOMBINÉ, VECTEUR D'EXPRESSION RECOMBINÉ, PROCÉDÉ DE PRÉPARATION CORRESPONDANT ET UTILISATION ASSOCIÉE

(30) Priority: 27.05.2019 CN 201910445910
(43) Date of publication of application: 29.09.2021
(73) Proprietor: Shenzhen Yhlo Biotech Co., Ltd, 518116 Shenzhen (CN)
(72) Inventor: ZHENG, Liang, Shenzhen, Guangdong 518116 (CN); LUO, Chunlei, Shenzhen, Guangdong 518116 (CN); QIAN, Chungen, Shenzhen, Guangdong 518116 (CN); HU, Kunhui, Shenzhen, Guangdong 518116 (CN)
(74) Representative: Michalski Hüttermann & Partner Patentanwälte mbB
(86) International application number: PCT/CN2019/127787
(87) International publication number: WO 2020/238181

(56) References cited:
- EP-A1- 1 672 368
- WO-A1-2011/112792
- WO-A1-2016/054038
- CN-A- 101 688 865
- CN-A- 101 838 327
- CN-A- 110 128 549
- MURCH SIMON ED - NAIR M K C ET AL: "Recent Advances in Celiac Disease", INDIAN JOURNAL OF PEDIATRICS, SPRINGER INDIA, NEW DELHI, vol. 83, no. 12, 8 June 2016 (2016-06-08), pages 1428 - 1435, XP036113324, ISSN: 0019-5456, [retrieved on 20160608], DOI: 10.1007/S12098-016-2161-8
- KAUR ANANTDEEP ET AL: "Celiac disease: from etiological factors to evolving diagnostic approaches", JOURNAL OF GASTROENTERLOGY, SPRINGER JAPAN KK, JP, vol. 52, no. 9, 19 June 2017 (2017-06-19), pages 1001 - 1012, XP036303670, ISSN: 0944-1174, [retrieved on 20170619], DOI: 10.1007/S00535-017-1357-7
- HANNA BRAGDE ET AL: "Potential blood-based markers of celiac disease", BMC GASTROENTEROLOGY, BIOMED CENTRAL LTD., LONDON, GB, vol. 14, no. 1, 9 October 2014 (2014-10-09), pages 176, XP021199134, ISSN: 1471-230X, DOI: 10.1186/1471-230X-14-176
- RAUHAVIRTA TIINA ET AL: "Transglutaminase 2 and Transglutaminase 2 Autoantibodies in Celiac Disease: a Review", CLINICAL REVIEWS IN ALLERGY AND IMMUNOLOGY, HUMANA PRESS, TOTOWA, NJ, US, vol. 57, no. 1, 4 June 2016 (2016-06-04), pages 23 - 38, XP037917562, ISSN: 1080-0549, [retrieved on 20160604], DOI: 10.1007/S12016-016-8557-4
- KALLIOKOSKI SUVI ET AL: "Transglutaminase 2-specific coeliac disease autoantibodies induce morphological changes and signs of inflammation in the small-bowel mucosa of mice", AMINO ACIDS, SPRINGER VERLAG, AU, vol. 49, no. 3, 9 August 2016 (2016-08-09), pages 529 - 540, XP036181973, ISSN: 0939-4451, [retrieved on 20160809], DOI: 10.1007/S00726-016-2306-0
- MCALLISTER BRIAN P ET AL: "A Comprehensive Review of Celiac Disease/Gluten-Sensitive Enteropathies", CLINICAL REVIEWS IN ALLERGY AND IMMUNOLOGY, HUMANA PRESS, TOTOWA, NJ, US, vol. 57, no. 2, 2 June 2018 (2018-06-02), pages 226 - 243, XP037044421, ISSN: 1080-0549, [retrieved on 20180602], DOI: 10.1007/S12016-018-8691-2
- HANSEN, H. ET AL.: "The Intestinal T Cell Response to Disease Is Focused on a Single Deamidated Glutamine Targeted by Tissue Transglutaminase", J. EXP. MED., vol. 191, no. 4, 21 February 2000 (2000-02-21), XP000986723, DOI: 20200311124648A

## Description

### TECHNICAL FIELD

The present disclosure relates to the technical field of diagnosis of celiac disease, in particular to a recombinant deamidated gliadin polypeptide antigen, a recombinant antigen-expressing gene, a recombinant expression vector, and a preparation method and use thereof.

### BACKGROUND

Celiac disease (CD), also known as idiopathic steatorrhea, adult celiac disease, non-tropical sprue, or gluten-induced enteropathy, is a chronic autoimmune enteropathy induced in genetically susceptible individuals due to the intake of gluten-containing grains (such as wheat, barley, and bare wheat) and their products. Studies on genes genetically related to celiac disease have shown that this disease is closely related to specific human leukocyte antigen class II genes, such as the HLA-DQ2 and HLA-DQ8 genes located in chromosome 6p21.

The diagnostic methods for celiac disease include genetic testing, intestinal biopsy, and serological testing. The genetic testing is mainly used to screen a high-risk population having HLA-DQ2 and HLA-DQ8 genotypes. However, due to being influenced by the environment, only 3% of people with these two genotypes would develop celiac disease. The intestinal biopsy is the gold criteria for diagnosing celiac disease. Positive small intestine biopsy is a diagnostic criteria for celiac disease recommended by the World Gastroenterology Organization, and a definite diagnosis can be made by a positive serological test. The serological testing is based on IgA (immunoglobulin A), including outdated IgA anti-gliadin antibody (AGA), subsequent IgA anti-endomysial antibody (anti-EMA) and IgA anti-tissue transglutaminase antibody (anti-tTG), but false-negative results for serum anti-tTG and anti-EMA may occur in patients with IgA deficiency. IgG-DGP, due to its high sensitivity and specificity, can be used as the most accurate testing means for patients with selective IgA deficiency. DGP antibodies, especially in babies whose immune system is not yet fully developed, allows earlier diagnosis of celiac disease than anti-tTG and anti-EMA. High concentrations of DGP antibodies are positively correlated with the severity of intestinal damage.

### SUMMARY

Accordingly, it is necessary to provide a recombinant deamidated gliadin polypeptide antigen with good sensitivity and high specificity that can be used for the diagnosis of celiac disease.

A recombinant deamidated gliadin polypeptide antigen comprises an interleukin 15 protein, and a DGP-1 peptide segment and a DGP-2 peptide segment linked to both ends of the interleukin 15 protein, respectively, the DGP-1 peptide segment having an amino acid sequence as set forth in SEQ ID NO. 1, and the DGP-2 peptide segment having an amino acid sequence as set forth in SEQ ID NO. 2.

In an embodiment, the recombinant deamidated gliadin polypeptide antigen further comprises a hexahistidine tag and a Flag tag. The interleukin 15 protein and the DGP-1 peptide segment and the DGP-2 peptide segment linked to the both ends of the interleukin 15 protein, respectively, form a fusion protein, and the hexahistidine tag and the Flag tag are linked to both ends of the fusion protein, respectively.

In an embodiment, the hexahistidine tag, the DGP-1 peptide segment, the interleukin-15 protein, the DGP-2 peptide segment, and the Flag tag are linked in sequence via a linker peptide.

The present disclosure provides a recombinant antigen-expressing gene comprising a nucleotide sequence corresponding to an amino acid sequence of the recombinant deamidated gliadin polypeptide antigen.

The present disclosure provides a recombinant expression vector comprising the recombinant antigen-expressing gene as described above and an expression vector.

In an embodiment, the expression vector is pET-21a(+), pET-28a(+) or pET-30a(+).

The present disclosure also provides use of the recombinant deamidated gliadin polypeptide antigen, the recombinant antigen-expressing gene or the recombinant expression vector as described above in preparing a product for diagnosing celiac disease.

The present disclosure also provides a kit for diagnosing celiac disease, comprising the recombinant deamidated gliadin polypeptide antigen as described above.

In an embodiment, the kit further comprises magnetic microspheres. The recombinant deamidated gliadin polypeptide antigen is conjugated to the magnetic microspheres having amino groups.

The present disclosure further provides a method for preparing the recombinant deamidated gliadin polypeptide antigen as described above, comprising: obtaining a nucleotide sequence corresponding to an amino acid sequence of the recombinant deamidated gliadin polypeptide antigen; double-digesting and linking the nucleotide sequence for insertion into an expression vector to obtain a recombinant expression vector; transforming the recombinant expression vector into a host cell for expression; and isolating and purifying the expression product to obtain the recombinant deamidated gliadin polypeptide antigen.

Two dominant DGP epitope peptide segments, namely the DGP-1 peptide segment and the DGP-2 peptide segment, were selected for the recombinant deamidated gliadin polypeptide antigen of the present disclosure, which is beneficial to improve the sensitivity and specificity of test. Furthermore, the DGP-1 peptide segment and DGP-2 peptide segment are linked to the both ends of the interleukin 15 protein, respectively. On the one hand, the interleukin 15 protein is a human endogenous protein without immunogenicity and thus background noise can be effectively reduced and false positives can be avoided. On the other hand, since the interleukin 15 protein itself is related to the reaction process of immune system against celiac disease, when some large peptide segments generated by the degradation of gluten penetrate the epithelial barrier and enter the lamina propria of the mucosa, the glutamine in these peptide segments can be deamidated by tissue transglutaminase to generate negatively charged glutamic acid, and these peptides then become deamidated gliadin polypeptides which thus have increased affinity to HLA-DQ2 and HLA-DQ8 on the surface of antigen-presenting cells, thereby enabling easy recognizability of these peptides by antigen-presenting cells and release of cytokine IL-15 from activated reactive CD4+ T cells, and consequently leading to a higher concentration of IL-15 in the serum in patients with celiac disease. Therefore, the fusion of the IL-15 to the DGP-1 peptide segment and the DGP-2 peptide segment is more conducive to improvement of the sensitivity and specificity of tests.

### BRIEF DESCRIPTION OF THE DRAWINGS

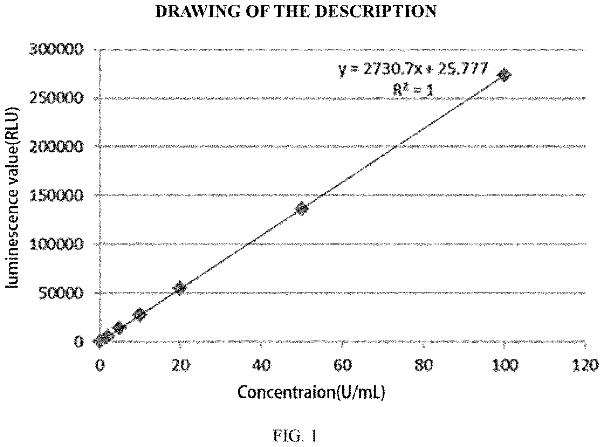

Fig. 1 is a standard curve obtained by detecting a deamidated gliadin calibrator samples in Example 1.

### DETAILED DESCRIPTION OF THE EMBODIMENTS

In order to understand the present disclosure, the present disclosure will be described more fully hereinafter, and preferred embodiments of the present disclosure will be given. The present disclosure invention may, however, be embodied in many different forms and should not be construed as limited to the embodiments set forth herein. Rather, these embodiments are provided so that this disclosure will be thorough and complete.

All technical and scientific terms used herein have the same meaning as commonly understood by those skilled in theart to which this invention belongs unless otherwise defined. The terms used in the description herein are only for the purpose of describing specific embodiments, and are not intended to limit the present disclosure. The term "and/or" as used herein includes any and all combinations of one or more associated listed items.

In an embodiment of the present disclosure, the recombinant deamidated gliadin polypeptide antigen comprises an interleukin 15 protein, and a DGP-1 peptide segment and a DGP-2 peptide segment linked to both ends of the interleukin 15 protein, respectively, the DGP-1 peptide segment having an amino acid sequence as set forth in SEQ ID NO. 1, the DGP-2 peptide segment having an amino acid sequence as set forth in SEQ ID NO. 2, and the interleukin 15 protein having an amino acid sequence as set forth in SEQ ID NO. 3. It can be understood that the positions of the DGP-1 peptide segment and the DGP-2 peptide segment can be interchanged, that is, the DGP-1 peptide segment can be linked to the N-terminus or C-terminus of the interleukin 15 protein.

Deamidated gliadin polypeptide (DGP) is a polypeptide which is obtained by absorbing and tTG (tissue transglutaminase)-modifying original peptide fragments of prolamin in cereals that are not completely hydrolyzed due to the abundance of proline, so as to deamidate glutamine residues at certain positions into glutamate residues. DGP stimulates the immune system to produce autoantibodies against DGP. The original polypeptide of DGP can be derived from α/β-gliadin, or from γ- or ω-gliadin. IgG-DGP, due to its high sensitivity and specificity, can be used as the most accurate testing method for patients with selective IgA deficiency. Especially in babies whose immune system is not yet fully developed, DGP antibodies allows earlier diagnosis of celiac disease than anti-tTG and anti-EMA. High concentrations of DGP antibodies are positively correlated with the severity of intestinal damage.

Currently, polypeptides are mainly synthesized by a chemical method and a recombinant expression method. The chemical method, namely solid phase polypeptide synthesis (SPPS), is a method for synthesizing peptides having predetermined amino acid sequences through chemical reactions, comprising: first, covalently attaching an amino acid whose amino group is protected by a blocking group to chloromethyl polystyrene resin serving as a solid-phase carrier; then, removing the protecting group of the amino group with trifluoroacetic acid; next, activating a carboxyl group of a second amino acid whose amino group is protected by N,N'-dicyclohexylcarbodiimide (DCC), and then reacting with the amino group of the first amino acid attached to the solid-phase carrier to form a peptide bond; repeating as above; and finally, removing the protecting group and hydrolyzing the ester bond between the peptide chain and the solid-phase carrier, resulting a predetermined polypeptide on the solid-phase carrier. However, the chemically synthesized polypeptides have low stability due to their strong hydrophobicity and lose their natural conformation, resulting in low sensitivity and high cost for detecting antibodies. According to the recombinant expression method, the target peptide segment is fused to some common tags such as Glutathione-S transferase (GST) and Maltose-binding protein (MBP), and the like, for expression. However, as foreign proteins, the tag proteins in these recombinant proteins are also immunogenic. Thus, these recombinant proteins are easy to cause strong background noise and even false positives when used as an antigen for detecting antibodies.

Two dominant DGP epitope peptide segments, namely the DGP-1 peptide segment and the DGP-2 peptide segment, were selected for the recombinant deamidated gliadin polypeptide antigen of the present disclosure, which is beneficial to improve the sensitivity and specificity of test. Furthermore, the DGP-1 peptide segment and DGP-2 peptide segment are linked to the both ends of the interleukin 15 protein, respectively. On the one hand, the interleukin 15 protein is a human endogenous protein without immunogenicity and thus background noise can be effectively reduced and false positives can be avoided. On the other hand, since the interleukin 15 protein itself is related to the reaction process of immune system against celiac disease, when some large peptide segments generated by the degradation of gluten penetrate the epithelial barrier and enter the lamina propria of the mucosa, the glutamine in these peptide segments can be deamidated by tissue transglutaminase to generate negatively charged glutamic acid and these peptides then become deamidated gliadin polypeptides which thus have increased affinity to HLA-DQ2 and HLA-DQ8 on the surface of antigen-presenting cells, thereby enabling easy recognizability of these peptides by antigen-presenting cells and release of cytokine IL-15 from activated reactive CD4+ T cells, and consequently leading to a higher concentration of IL-15 in the serum in patients with celiac disease. Therefore, the fusion of the IL-15 to the DGP-1 peptide segment and the DGP-2 peptide segment is more conducive to improvement of the sensitivity and specificity of tests.

In summary, the recombinant deamidated gliadin polypeptide antigen provided in the present disclosure has good sensitivity, high specificity, and low preparation cost in the diagnosis of celiac disease, allows determination of the IgG-DGP antibody in serum, especially enables diagnosis for potential patients with celiac disease among patients with IgA deficiency and infant population, effectively compensates for the shortcomings of anti-EMA and anti-tTG, reduces the risk of false positives, and is very suitable for large-scale screening for celiac disease. It can be understood that the recombinant deamidated gliadin polypeptide antigen as described above can be applied to the determination of IgG-DGP antibodies as well as the determination of IgA-DGP antibodies.

In a specific example, the recombinant deamidated gliadin polypeptide antigen further comprises a hexahistidine tag and a Flag tag, wherein the interleukin 15 protein and the DGP-1 peptide segment and the DGP-2 peptide segment linked to the both ends of the interleukin 15 protein, respectively, form a fusion protein, and the hexahistidine tag and the Flag tag are linked to both ends of the fusion protein, respectively. It can be understood that the positions of the hexahistidine tag and the Flag tag can be interchanged as long as they are located at the terminus ends. For example, when the DGP-1 peptide segment is linked to the N-terminus of the interleukin 15 protein, the hexahistidine tag is linked to the N-terminus of the DGP-1 peptide segment, and the Flag tag is linked to the C-terminus of the DGP-2 peptide segment. The hexahistidine tag has an amino acid sequence as set forth in SEQ ID NO. 4, and the Flag tag has an amino acid sequence as set forth in SEQ ID NO. 5. In this way, on the one hand, the hexahistidine tag and the Flag tag can facilitate the separation and purification of the fusion protein, on the other hand, the hexahistidine tag and the Flag tag can also increase the hydrophilicity of the fusion protein, thereby contributing to the improvement of water solubility of the fusion protein and stabilization of the natural conformation of the fusion protein, and enhancing the sensitivity and stability during detection. It can be understood that other tag sequences can also be selected as required.

In a specific example, the hexahistidine tag, the DGP-1 peptide segment, the interleukin-15 protein, the DGP-2 peptide segment, and the Flag tag are linked in sequence via a linker peptide. The linker peptide has an amino acid sequence set forth in SEQ ID No.6. The recombinant deamidated gliadin polypeptide antigen of this specific example has an amino acid as set forth in SEQ ID NO. 7. It can be understood that, depending on different expression vectors or hosts, additional amino acids may be added at both ends of the amino acid sequence. For example, a methionine translated from an initiation codon may be retained if there is no processing modification after expression in the host.

In an embodiment of the present disclosure, the recombinant antigen-expressing gene comprises a nucleotide sequence corresponding to the amino acid sequence of the recombinant deamidated gliadin polypeptide antigen. It can be understood that there are various alternates of the corresponding nucleotide sequence according to the diversity of codons.

In a specific example, the recombinant antigen-expressing gene has a nucleotide sequence as set forth in SEQ ID NO. 8. The nucleotide sequence is a DNA sequence codon-optimized for expression in E. coli, which has a good expression effect. It can be understood that in order to facilitate the enzyme digestion and the ligation to the expression vector, the sequence has a NdeI site upstream and a HindIII site downstream, respectively. In other specific examples, the upstream and downstream nucleotide sequences can be adjusted according to the required digestion site.

In an embodiment of the present disclosure, a recombinant expression vector comprises the recombinant antigen-expressing gene as described above and an expression vector.

In a specific example, the expression vector is pET-21a(+), pET-28a(+) or pET-30a(+). It can be understood that the expression vector is not limited to this, and other plasmid vectors or viral vectors can be selected as required.

In an embodiment of the present disclosure, provided is use of the recombinant deamidated gliadin polypeptide antigen, the recombinant antigen-expressing gene or the recombinant expression vector as described above in preparing a product for diagnosing celiac disease, wherein the product for diagnosing celiac disease is a kit for diagnosing celiac disease.

In an embodiment of the present disclosure, the kit for diagnosing celiac disease comprises the recombinant deamidated gliadin polypeptide antigen as described above. The kit for diagnosing celiac disease using the recombinant deamidated gliadin polypeptide antigen as described above can be used for determining DGP antibodies by chemiluminescent microparticle immunoassay (CMIA) and has advantages such as high sensitivity, high specificity, good repeatability, low cost, and the like.

In a specific example, the kit for diagnosing celiac disease further comprises magnetic microspheres. The recombinant deamidated gliadin polypeptide antigen is conjugated to the magnetic microspheres having amino groups. Thus, the magnetic microspheres can be conjugated to the recombinant deamidated gliadin polypeptide antigen by chemical crosslinking.

In an embodiment of the present disclosure, a method for preparing the recombinant deamidated gliadin polypeptide antigen as described above comprises: obtaining a nucleotide sequence corresponding to an amino acid sequence of the recombinant deamidated gliadin polypeptide antigen as described above; double-digesting and linking the nucleotide sequence for insertion into an expression vector to obtain a recombinant expression vector; transforming the recombinant expression vector into a host cell for expression; and isolating and purifying the expression product, thereby obtaining the recombinant deamidated gliadin polypeptide antigen.

In a specific example, the nucleotide sequence corresponding to the amino acid sequence of the recombinant deamidated gliadin polypeptide antigen can be obtained by gene synthesis. The host can be selected according to the type of expression vectors, such as E. coli BL21 (DE3) strain and the like.

In the following, the present disclosure will be further described in detail principally in combination with specific embodiments and drawings.

### Example 1

### 1. Preparation of recombinant deamidated gliadin polypeptide antigen

The following were selected, dominant DGP epitope peptide segments having amino acid sequences of SEQ ID NO.1 (named DGP-1 peptide segment) and SEQ ID NO.2 (named DGP-2 peptide segment), IL-15 protein having an amino acid sequence of SEQ ID NO.3, the hexahistidine tag having an amino acid sequence of SEQ ID NO.4, the Flag tag having an amino acid sequence of SEQ ID NO.5, and the linker peptide having an amino acid sequence of SEQ ID NO.6. The hexahistidine tag, the DGP-1 peptide segment, the interleukin 15, the DGP-2 peptide segment and the Flag tag were all linked via a linker peptide and fused into a recombinant DGP antigen having a sequence of SEQ ID NO.7. The DNA of the recombinant DGP antigen having a sequence of SEQ ID NO. 8 was obtained by gene synthesis (General Biosystems (Anhui) Co., Ltd.). The DNA of the DGP recombinant antigen has a *Nde*I site upstream and a *Hind*III site downstream. The DNA was digested with a corresponding restriction endonuclease and ligated to the expression vector pET-21a(+) digested with *Nco*I and *Hind*III, obtaining a recombinant plasmid pET-21a(+)-DGP.

The recombinant plasmid was transformed into E. coli expression strain BL21(DE3). A single colony was picked and inoculated into 20 mL of LB medium containing 100 µg/mL ampicillin, and incubated overnight at 37°C and 200 rpm. The next day, 1%vol of the overnight culture was pipetted into 1 L of fresh LB medium containing 100 µg/mL ampicillin, and then incubated at 37°C and 200 rpm until OD₆₀₀ reached about 0.6. The culture was induced by IPTG (isopropylthiogalactoside) at a final concentration of 1 mM at 18°C for 20 hours for expression. The cells were collected by centrifugation at 10000 g and at 4°C for 3 mins, suspended in 40 mL of 50 mM Tris 8.0/500 mM NaCl buffer solution (buffer A) pre-cooled on ice per liter of liquid culture. After disrupted with a high-pressure homogenizer, the resultant substance was centrifuged at 12000 g and at 4°C for 30 mins. The supernatant was filtered using a 0.22 µm filter membrane and passed through a nickel column. After the nickel column was equilibrated with 10 column volumes of buffer A. the supernatant was loaded. Unbound proteins were washed off with 10 column volumes of buffer A. Then, impure proteins were washed off with 10 column volumes of 50 mM Tris 8.0/500 mM NaCl/60 mM imidazole buffer (buffer B). Next, the target protein was eluted with a gradient buffer containing 60 mM-500 mM imidazole. The target protein with a purity of 90% was selected, and dialyzed at 4°C in a PBS buffer containing 20% glycerol, and stored at -20°C for later use.

### 2. Preparation of kit for diagnosing celiac disease

### (1) Preparation of magnetic nanobeads coated with the recombinant deamidated gliadin polypeptide antigen

50 mg of suspension of carboxylated magnetic particles (having a particle size in a range from 0.05 µm to1 µm) was separated magnetically, and the sediment was retained, and then resuspended in 20 mM MES buffer at pH 5.5. 1 mL of 10 mg/mL EDC aqueous solution prepared freshly was added, so as to activate the carboxyl groups on the surface of the magnetic beads. 4 mg of recombinant DGP antigen was added, and the resultant mixture was suspended at room temperature for 6 h and magnetically separated. The supernatant was removed. The magnetic particles were resuspended at 1 mg/mL in 100 mM Tris buffer at pH 8.0 containing 2% BSA, to obtain coated magnetic particles, which were divided into 5 mL/bottle and stored at 4°C for later use.

### (2) Preparation of mouse anti-human IgG labeled acridinium ester:

To 50 µL of 25 mg/mL mouse anti-human IgG, 150 µL of 0.1 M carbonate buffer at pH 9.0 to 9.5 was added and mixed well. Then, 1.5 µL of 5 mg/mL acridinium ester was added and mixed well. The resultant mixture reacted in the dark at room temperature for1.5 h and then taken out for desalting in a 5 mL GE Desalting prepacked column. The chromatography column was equilibrated using TBS, and then the reacted acridinium ester solution was added. The protein samples at the peak were collected, divided into 5 mL/bottle and stored at 4°C for later use.

### (3) Preparation of deamidated gliadin calibrator samples

Deamidated gliadin IgG antibody was formulated with buffer (40 mM Tris-Cl, 0.5% BSA, 1% NaCl, pH 8.0) into solutions at a concentration of 0 U/mL, 2 U/mL, 5 U/mL, 10 U/mL, 20 U/mL, 50 U/mL, 100 U/mL,. The deamidated gliadin calibrator samples were divided into 5 mL/bottle, lyophilized and stored at 4°C for later use.

The deamidated gliadin calibrator samples as above were tested using a two-step indirect chemiluminescence method to plot a standard curve as shown in FIG. 1. Then an actual sample was tested to calculate the concentration of the sample based on the luminescence value of the sample.

Sensitivity testing:
The sensitivity of the kit for diagnosing celiac disease as above was calculated with reference to the experimental protocol recommended by CLSI EP17-A, giving the result of 2.0 U/mL.

### Linearity testing:

A linear analysis was performed for the standard samples at a concentration of 0 U/mL, 2 U/mL, 5U/mL, 10 U/mL, 20 U/mL, 50 U/mL, and 100 U/mL to calculate a linear correlation coefficient (r=0.9996). Additionally, the kit is used to detect the deamidated gliadin antibody samples in a linear range from 0 U/mL to 100 U/mL.

### Precision testing:

Both the deamidated gliadin antibody samples at concentrations of 20 U/mL and 100 U/mL were tested in triplicate using three batches of kits, to calculate intra-batch and inter-batch differences among the kits. The result showed that both the intra-batch and inter-batch differences were less than 5%.

### Interference experiment:

To mixed serum, each of the interfering substances, i.e. conjugated bilirubin, free bilirubin, hemoglobin, ascorbic acid, and glycerides, was added respectively in a mass ratio of 1:20. The mixed serum with and without the various interfering substances were tested to calculate the deviation therebetween. Deviation within ±10% was regarded as an acceptable range. The results showed that the interference meets the criteria in the NCCLS document. This method can be used for an accurate assessment of the status of deamidated gliadin antibodies in clinical laboratories.

### Comparative Example 1

The method of this Comparative Example was substantially the same as that of Example 1, except that the interleukin 15 was replaced with SUMO. Correspondingly, the prepared kit has a sensitivity of 2.0 U/mL. The intra-batch and inter-batch differences of the kit were CV=4.15%, and CV=16.25%, respectively. The interference did not meet the criteria in the NCCLS document.

### Comparative Example 2

The method of this Comparative Example was substantially the same as that of Example 1, except that the DGP-1 peptide segment was directly connected with the DGP-2 peptide segment without using the intermediate interleukin 15. Correspondingly, the prepared kit has a sensitivity of 1.8 U/mL. The intra-batch and inter-batch differences of the kit were CV=5.4% and CV=17.36%, respectively. The interference did not meet the criteria in the NCCLS document.

### Comparative Example 3

The method of this Comparative Example was substantially the same as that of Example 1, except that the DGP-1 peptide is replaced with another DGP epitope peptide having an amino acid sequence as follows: QPEQPQQSFPEQERPF. Correspondingly, the prepared kit has a sensitivity of 2.4 U/mL. The intra-batch and inter-batch differences of the kit were CV=4.15%, and CV=16.25%, respectively. The interference did not meet the criteria in the NCCLS document.

### Example 2

The method of this Example was substantially the same as that of Example 1, except that pET-28a(+) was selected as the expression vector. Correspondingly, the prepared kit has a sensitivity of 0.8 U/mL. The intra-batch and inter-batch differences of the kit were CV=3.65%, and CV=4.87%, respectively. The interference met the criteria in the NCCLS document.

The technical features of the above-mentioned embodiments can be combined arbitrarily. In order to make the description concise, all of the possible combinations of the various technical features in the foregoing embodiments are not described. However, the combination of these technical features should be considered within the scope of this specification, as long as they have no collision with each other.

## Claims

1. A recombinant deamidated gliadin polypeptide antigen, comprising an interleukin 15 protein, and a DGP-1 peptide segment and a DGP-2 peptide segment linked to both ends of the interleukin 15 protein, respectively, the DGP-1 peptide segment having an amino acid sequence as set forth in SEQ ID NO. 1, and the DGP-2 peptide segment having an amino acid sequence as set forth in SEQ ID NO. 2.

2. The recombinant deamidated gliadin polypeptide antigen according to claim 1, further comprising a hexahistidine tag and a Flag tag, wherein the interleukin 15 protein and the DGP-1 peptide segment and the DGP-2 peptide segment linked to the both ends of the interleukin 15 protein, respectively, form a fusion protein, and the hexahistidine tag and the Flag tag are linked to both ends of the fusion protein, respectively.

3. The recombinant deamidated gliadin polypeptide antigen according to claim 2, wherein the hexahistidine tag, the DGP-1 peptide segment, the interleukin-15 protein, the DGP-2 peptide segment, and the Flag tag are linked in sequence via a linker peptide.

4. A recombinant antigen-expressing gene comprising a nucleotide sequence corresponding to an amino acid sequence of the recombinant deamidated gliadin polypeptide antigen according to any one of claims 1 to 3.

5. A recombinant expression vector comprising the recombinant antigen-expressing gene according to claim 4 and an expression vector.

6. The recombinant expression vector according to claim 5, wherein the expression vector is pET-21a(+), pET-28a(+) or pET-30a(+).

7. Use of the recombinant deamidated gliadin polypeptide antigen according to any one of claims 1 to 3, the recombinant antigen-expressing gene according to claim 4 or the recombinant expression vector according to any one of claims 5 to 6 in preparing a product for diagnosing celiac disease.

8. A kit for diagnosing celiac disease, comprising the recombinant deamidated gliadin polypeptide antigen according to any one of claims 1 to 3.

9. The kit for diagnosing celiac disease according to claim 8, further comprising magnetic microspheres, wherein the recombinant deamidated gliadin polypeptide antigen is conjugated to the magnetic microspheres having amino groups.

10. A method for preparing the recombinant deamidated gliadin polypeptide antigen according to any one of claims 1 to 3, comprising: obtaining a nucleotide sequence corresponding to an amino acid sequence of the recombinant deamidated gliadin polypeptide antigen; double-digesting and linking the nucleotide sequence for insertion into an expression vector to obtain a recombinant expression vector; transforming the recombinant expression vector into a host cell for expression; and isolating and purifying the expression product to obtain the recombinant deamidated gliadin polypeptide antigen.

## Patentansprüche

1. Ein rekombinantes deamidiertes Gliadin-Polypeptid-Antigen, umfassend ein Interleukin-15-Protein und ein DGP-1-Peptidsegment und ein DGP-2-Peptidsegment, die jeweils an beide Enden des Interleukin-15-Proteins gebunden sind, wobei das DGP-1-Peptidsegment die Aminosäuresequenz SEQ ID NO. 1 hat und das DGP-2-Peptidsegment die Aminosäuresequenz SEQ ID NO. 2 hat.

2. Das rekombinante deamidierte Gliadin-Polypeptid-Antigen nach Anspruch 1, umfassend einen Hexahistidin-Tag und einen Flag-Tag, wobei das Interleukin-15-Protein und das DGP-1-Peptidsegment und das DGP-2-Peptidsegment, die jeweils an beide Enden des Interleukin-15-Proteins gebunden sind, wobei das DGP-1-Peptidsegment, ein Fusionsprotein bilden und der Hexahistidin-Tag und der Flag-Tag jeweils an beide Enden des Fusionsproteins gebunden sind.

3. Das rekombinante deamidierte Gliadin-Polypeptid-Antigen nach Anspruch 2, wobei der Hexahistidin-Tag, das DGP-1-Peptidsegment, das Interleukin-15-Protein, das DGP-2-Peptidsegment und der Flag-Tag in der Sequenz über ein Linker-Peptid verbunden sind.

4. Ein rekombinantes Antigen-exprimierendes Gen, umfassend eine Nukleotidsequenz, die einer Aminosäuresequenz des rekombinanten deamidierten Gliadin-Polypeptid-Antigens nach einem der Ansprüche 1 bis 3 entspricht.

5. Ein rekombinanter Expressionsvektor, umfassend das rekombinante Antigen-exprimierende Gen nach Anspruch 4 und einen Expressionsvektor.

6. Der rekombinante Expressionsvektor nach Anspruch 5, wobei der Expressionsvektor pET-21a(+), pET-28a(+) oder pET-30a(+) ist.

7. Das rekombinante deamidierte Gliadin- Polypeptid-Antigen nach einem der Ansprüche 1 bis 3, das rekombinante Antigen-exprimierende Gen nach Anspruch 4 oder der rekombinante Expressionsvektor nach einem der Ansprüche 5 bis 6 zur Verwendung bei der Diagnose von Zöliakie.

8. Kit zur Diagnose von Zöliakie, umfassend das rekombinante deamidierte Gliadin-Polypeptid-Antigen nach einem der Ansprüche 1 bis 3.

9. Kit zur Diagnose von Zöliakie nach Anspruch 8, ferner umfassend magnetische Mikrokugeln, wobei das rekombinante deamidierte Gliadin-Polypeptid-Antigen an die magnetischen Mikrokugeln mit Aminogruppen konjugiert ist.

10. Verfahren zur Herstellung des rekombinanten deamidierten Gliadin-Polypeptid-Antigens nach einem der Ansprüche 1 bis 3, umfassend: Erhalten einer Nukleotidsequenz, die einer Aminosäuresequenz des rekombinanten deamidierten Gliadin-Polypeptid-Antigens entspricht; Doppelverdau und Verknüpfen der Nukleotidsequenz zur Insertion in einen Expressionsvektor um einen rekombinanten Expressionsvektor zu erhalten; Transformieren des rekombinanten Expressionsvektors in eine Wirtszelle zur Expression, um ein Expressionsprodukt zu erhalten; und Isolieren und Reinigen des Expressionsprodukts, um das rekombinante deamidierte Gliadin-Polypeptid-Antigen zu erhalten.

## Revendications

1. Antigène polypeptidique de gliadine désaminée recombiné, comprenant une protéine interleukine 15, et un segment peptidique DGP-1 et un segment peptidique DGP-2 respectivement liés aux deux extrémités de la protéine interleukine 15, le segment peptidique DGP-1 ayant une séquence d'acides aminés telle que décrite dans SEQ ID n° 1, et le segment peptidique DGP-2 ayant une séquence d'acides aminés telle que décrite dans SEQ ID n° 2.

2. Antigène polypeptidique de gliadine désaminée recombiné selon la revendication 1, comprenant en outre une étiquette hexahistidine et une étiquette Flag, dans lequel la protéine interleukine 15 et les segments peptidiques DGP-1 et DGP-2 respectivement liés aux deux extrémités de la protéine interleukine 15 forment une protéine de fusion, et où l'étiquette hexahistidine et l'étiquette Flag sont respectivement liées aux deux extrémités de la protéine de fusion.

3. Antigène polypeptidique de gliadine désaminée recombiné selon la revendication 2, dans lequel l'étiquette hexahistidine, le segment peptidique DGP-1, la protéine interleukine 15, le segment peptidique DGP-2 et l'étiquette Flag sont liés en séquence via un peptide de liaison.

4. Gène exprimant un antigène recombiné, comprenant une séquence nucléotidique correspondant à une séquence d'acides aminés de l'antigène polypeptidique de gliadine désaminée recombiné selon l'une quelconque des revendications 1 à 3.

5. Vecteur d'expression recombiné comprenant le gène exprimant l'antigène recombiné selon la revendication 4 et un vecteur d'expression.

6. Vecteur d'expression recombiné selon la revendication 5, dans lequel le vecteur d'expression est pET-21a(+), pET-28a(+) ou pET-30a(+).

7. Utilisation de l'antigène polypeptidique de gliadine désaminée recombiné selon l'une quelconque des revendications 1 à 3, du gène exprimant l'antigène recombiné selon la revendication 4 ou du vecteur d'expression recombiné selon l'une quelconque des revendications 5 à 6 dans la préparation d'un produit pour diagnostiquer la maladie coeliaque.

8. Kit pour diagnostiquer la maladie cœliaque, comprenant l'antigène polypeptidique de gliadine désaminée recombiné selon l'une quelconque des revendications 1 à 3.

9. Kit pour diagnostiquer la maladie coeliaque selon la revendication 8, comprenant en outre des microsphères magnétiques, dans lequel l'antigène polypeptidique de gliadine désaminée recombiné est conjugué aux microsphères magnétiques ayant des groupes amino.

10. Procédé pour préparer l'antigène polypeptidique de gliadine désaminée recombiné selon l'une quelconque des revendications 1 à 3, comprenant : l'obtention d'une séquence nucléotidique correspondant à une séquence d'acides aminés de l'antigène polypeptidique de gliadine désaminée recombiné ; la double digestion et la liaison de la séquence nucléotidique pour insertion dans un vecteur d'expression pour obtenir un vecteur d'expression recombiné ; la transformation du vecteur d'expression recombiné dans une cellule hôte pour expression ; et l'isolement et la purification du produit d'expression pour obtenir l'antigène polypeptidique de gliadine désaminée recombiné.
